# EUROPEAN PATENT APPLICATION

(11) **EP 1 371 330 A1**
(43) Date of publication of application: **17.12.2003**
(21) Application number: 02700718.6
(22) Date of filing: 22.02.2002
(51) Int. Cl.: A61B 6/03

(54) **X-RAY CT APPARATUS AND X-RAY CT APPARATUS IMAGING METHOD**

(30) Priority: 23.02.2001 JP 2001048678; 13.07.2001 JP 2001214376
(71) Applicant: Mitsubishi Heavy Industries, Ltd., Tokyo 100-8315 (JP)
(72) Inventor: MIHARA, Kazumasa, c/o Mitsubishi Heavy Ind. Ltd., Hiroshima-shi (JP); HORI, Keiichi, Mitsubishi Heavy Ind. Ltd., Takasago-shi (JP); KAMINOU, Yuichiro, Mitsubishi Heavy Ind. Ltd., Nagoya-shi, Aichi 455-8515 (JP); OGURA, Shin, c/o MITSUBISHI HEAVY INDUSTRIES, LTD., Tokyo 100-8315 (JP)
(74) Representative: Henkel, Feiler, Hänzel
(86) International application number: JP0201620
(87) International publication number: WO02065916

(57) **Abstract**

An X-ray CT apparatus according to the present invention comprises a plurality of X-ray generating means which generate X-rays, surround a measuring object, are arranged on a circle with the measuring object at the center, and are relatively movable with respect to the measuring object, a detector which surrounds the measuring object, is arranged on a circle with the measuring object at the center, is relatively movable with respect to the measuring object, and detects X-rays emitted from the X-ray generating means and transmitted through the measuring object, and X-ray generation controlling means which selects use/non-use of each of the X-ray generating means, and a mode setting directing means for directing a desired operation mode with respect to the X-ray generation controlling means.

## Description

### Technical Field

The present invention relates to an X-ray CT apparatus which uses X-ray sources, has a fixed type detector, the X-ray sources being densely arranged on a concentric circle with respect to a measuring object, and relates to X-ray CT apparatus radiography.

### Background Art

An X-ray CT (Computed Tomography) apparatus is configured as follows. X-rays are emitted by sequentially varying the irradiation angle of the X-rays with respect to a measuring object. The X-rays transmitted through the measuring object are detected by a detection section. A tomogram of the measuring object is constituted by performing arithmetic operation processing based on the X-rays detected by the detection section.

In the simplified X-ray CT apparatus, an X-ray generation device which generates the X-rays and the detection section which detects the X-rays form a pair and are arranged so as to be symmetrically placed via the measuring object. The X-ray generation device and the detection section move relatively to the measuring object. In the X-ray CT apparatus, the X-ray generation device and the detection section rotate and move around the movement axis, and a plurality of tomograms are constituted by performing the radiography.

Further, there is an X-ray CT apparatus which can obtain a tomogram in a shorter time. In this X-ray CT apparatus, a plurality of X-ray generation devices which generate the X-rays by electron beams are aligned and fixedly arranged at even intervals on a circle (concentric circle) with the measuring object at the center. The X-ray CT apparatus performs control to sequentially electrically switch these X-ray generation devices adjacent to each other in the circumferential direction. The X-ray CT apparatus irradiates the measuring object with the X-rays and constitutes a tomogram image from information obtained in the detection portion.

The above-described X-ray CT apparatus is configured to obtain a tomogram by sequentially switching a plurality of X-ray sources in the circumferential direction. Therefore, a plurality of tomograms must be taken in order to obtain a necessary tomogram, and this takes time.

In the case of using the X-ray CT apparatus for an industrial purpose, a dose of the X-rays can be increased and radiography can be carried out in a short time. When using the X-ray CT apparatus for a medical purpose, however, necessary information must be obtained with the number of tomograms reduced as much as possible in order to suppress an exposed dose of a patient.

Further, the X-ray CT apparatus is essentially configured to obtain a tomogram of a measuring object. Therefore, in the case of obtaining a radiographic image of a measuring object from an arbitrary angle based on a tomogram, the X-ray radiographic apparatus must be used. It is often the case that a patient who needs a tomogram obtained by the X-ray CT apparatus has a serious case. It is not preferable for a patient of a serious case to make the switch from the X-ray CT apparatus to the X-ray radiographic apparatus every time image pickup is carried out.

In the X-ray radiographic apparatus, the measuring object must be inclined or the X-ray generation device must be inclined in order to take a radiogram from an arbitrary angle. When the X-ray radiographic apparatus is used for a medical purpose and a patient is inclined and held, the patient is troubled depending on an inclination angle and the patient is uncomfortable.

Furthermore, even if a direction of a desired image is determined by the X-ray CT apparatus, the angle adjusting must be again performed by making the switch to a different apparatus for the patient. At this moment, a position of the patient slightly deviates, and hence the radiography including preliminary images must be performed more than needed. Therefore, the exposed dose of the patient is also increased.

It is an object of the present invention to provide an X-ray CT apparatus and X-ray CT apparatus radiography capable of obtaining necessary examination information while suppressing the exposed does of a measuring object to the minimum level.

### Disclosure of the Invention

(1) According to the present invention, there is provided an X-ray CT apparatus comprising: a plurality of X-ray generating means which generate X-rays, surround a measuring object, are arranged on a circle with the measuring object at the center, and are relatively movable with respect to the measuring object; a detector which surrounds the measuring object, is arranged on a circle with the measuring object at the center, is relatively movable with respect to the measuring object, and detects X-rays emitted from the X-ray generating means and transmitted through the measuring object; X-ray generation controlling means for selecting use/non-use of each of the X-ray generating means; and mode setting directing means for directing a desired operation mode to the X-ray generation controlling means.
(2) According to the X-ray CT apparatus of the present invention defined in (1), plural lines of the detectors are provided in a direction of relative movement of the measuring object and the X-ray generation devices.
(3) According to the X-ray CT apparatus of the present invention defined in (1) or (2), the mode setting directing means has a single ray source radiography mode by which the X-ray generation controlling means is caused to switch one of the X-ray generating means and sequentially use them.
(4) 'According to the X-ray CT apparatus of the present invention defined in (1) or (2), the mode setting directing means has a multiple source synchronous radiography mode by which the X-ray generation controlling means is caused to simultaneously switch a plurality of the X-ray generating means distanced in the circumferential direction at predetermined intervals among a plurality of the X-ray generating means and sequentially use them.
(5) According to the X-ray CT apparatus of the present invention defined in (4), the multiple source synchronous radiography mode simultaneously uses a plurality of arbitrary X-ray generating means whose X-rays emitted therefrom do not overlap each other on the detectors.
(6) According to the X-ray CT apparatus of the present invention defined in (1) or (2), the mode setting directing means has a sector slice mode which causes the X-ray generation controlling means to switch the X-ray generating means forming a continuous part of the X-ray generating means group among a plurality of the X-ray generating means and sequentially use them.
(7) According to the X-ray CT apparatus of the present invention defined in (1) or (2), the mode setting directing means has a single shot mode which causes the X-ray generation controlling means to use one of the respective X-ray generating means for one time or simultaneously use a plurality of the X-ray generating means for one time.
(8) According to the present invention, there is provided an X-ray CT apparatus radiography comprising: obtaining information required for a tomogram of the measuring object by using the X-ray CT apparatus defined in any of (1) to (7); operating the X-ray generating means suitable for radiography of a specific part of the measuring object based on the tomogram constituted by this information; performing radiography of the measuring object without changing a posture of the measuring object.

### Brief Description of Drawings

FIG. 1 is a side cross-sectional view showing a schematic structure of an X-ray CT apparatus according to an embodiment of the present invention;
FIG. 2 is a cross-sectional view of a measurement device main body of the X-ray CT apparatus taken along the line F2-F2 in FIG. 1 according to the embodiment of the present invention;
FIG. 3 is a partial perspective view of the X-ray CT apparatus showing the positional relationship between an X-ray generation device and a detector according to the embodiment of the present invention;
FIGS. 4A and 4B are views showing a detailed structure of the X-ray generation device according to the embodiment of the present invention; and
FIG. 5 is a block diagram showing a control system of X-ray generation in the X-ray CT apparatus according to the embodiment of the present invention.

### Best Mode for Carrying Out of the Invention

A preferred embodiment according to the present invention will now be described hereinafter with reference to the accompanying drawings.

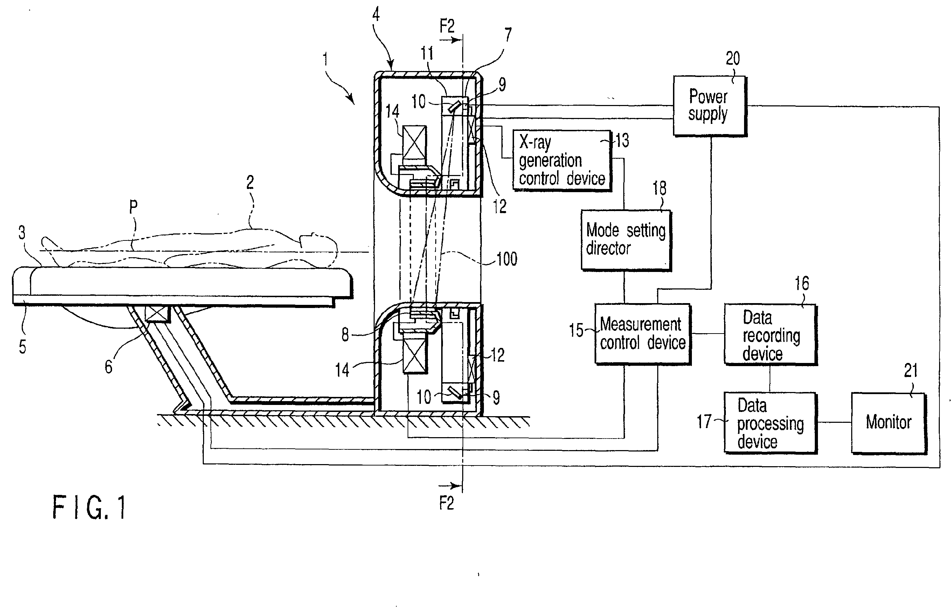

FIG. 1 is a side cross-sectional view showing a schematic structure of an X-ray CT apparatus according to the embodiment of the present invention. This X-ray CT apparatus is applied in a medical diagnosis field.

A high-speed X-ray CT apparatus 1 shown in FIG. 1 includes a bed 3 and a measurement device main body 4. A patient 2 as a measuring object is horizontally laid down in the bed 3. The bed 3 is attached to a slide 5 so as to move in a direction along which an axial line P running from the head to the toe of the patient 2 extends. The bed 3 horizontally reciprocates by a drive device 6. To the measurement device main body 4 is provided an opening through which the bed on which the patient 2 is laid can pass.

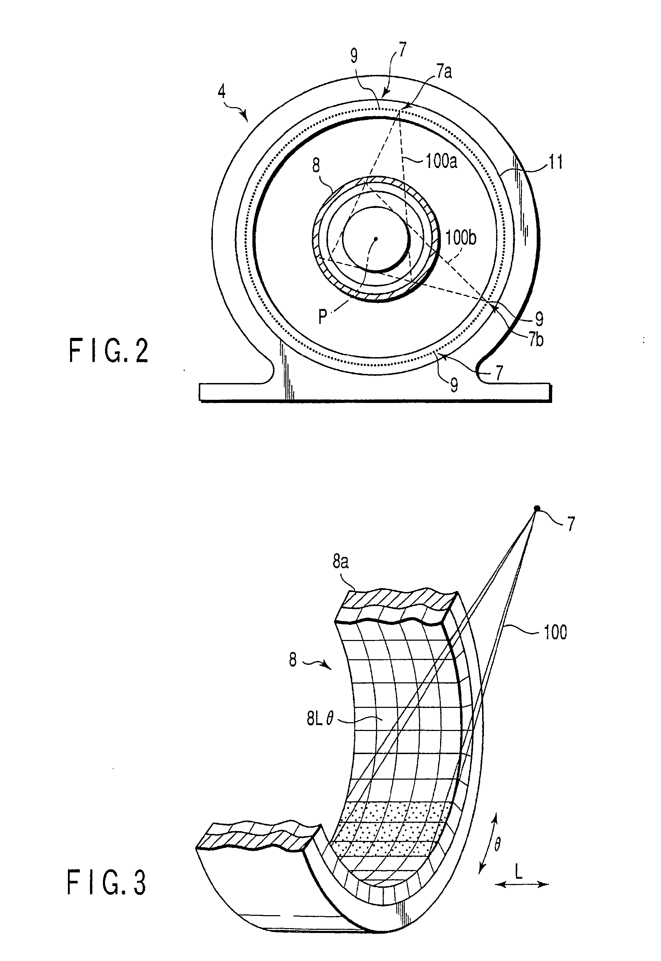

FIG. 2 is a front cross-sectional view (cross-sectional view taken along the line F2-F2 in FIG. 1) showing a schematic structure of the X-ray CT apparatus. As shown in FIG. 2, the measurement device main body 4 includes many (a plurality of) X-ray generation devices 7 and a detector 8. The many X-ray generation devices 7 are densely arranged in the circumferential direction of a concentric circle with an axial line P of the patient 2 at the center. The detector 8 detects X-rays 100 emitted from the X-ray generation devices 7 and transmitted through the patient 2.

FIG. 3 is a partial perspective view of the X-ray CT apparatus showing the physical relationship between the X-ray generation devices 7 and the detector 8. As shown in FIG. 3, the detector 8 has a ring 8a for a sensor array holder which is formed into a cylindrical shape with the axial line P at the center. Many sensor arrays 8_{Lθ} which detect the X-rays 100 are arranged on the inner surface of the ring 8a in such a manner that they are insulated from each other and densely aligned in a direction L along the axial line P and the circumferential direction θ. That is, the detector 8 is a so-called assembly of the sensor arrays 8_{Lθ}. The detector 8 is formed into a cylindrical shape having a predetermined width in the moving direction of the bed 3. Therefore, the X-ray generation devices 7 are arranged at positions which relatively deviate from the detector 8 in the direction along the axial line P. As a result, it is possible to emit the X-rays 100 which direct from the outside of the ring 8a toward the sensor arrays 8_{Lθ} on the inner side of the detector 8 by the X-ray generation devices 7.

It is to be noted that the detector 8 can be manufactured by accreting alloy based cadmium-tellurium (Cd-Te) on an insulator such as ceramic by deposition or plating and then dividing the obtained product into individual sensor arrays by, e.g., etching or laser processing. At this moment, the shape of each sensor array 8_{Lθ} seen from the inner surface may be a rectangular shape or a hexagonal shape. Incidentally, as to the conformation of the detector 8, it is good enough if it can efficiently and densely detect the X-rays, and the material or manufacturing method is not restricted to that described above.

Moreover, as shown in FIG. 1, each X-ray generation device 7 includes an electron gun 9 and a target 10 which receives an electron beam emitted from the electron gun 9 and generates the X-rays 100. In addition, as shown in FIG. 2, all the X-ray generation devices 7 are accommodated in a vacuum chamber 11 formed into an annular shape so as to cover the outer side of these devices. Additionally, to each electron gun 9 is connected an electron gun drive circuit 12 which controls emission of the electron beam as shown in FIG. 1. The timing for emitting the electron beam, i.e., the timing of emitting the X-rays 100 of each electron gun drive circuit 12 is controlled by an X-ray generation control device 13.

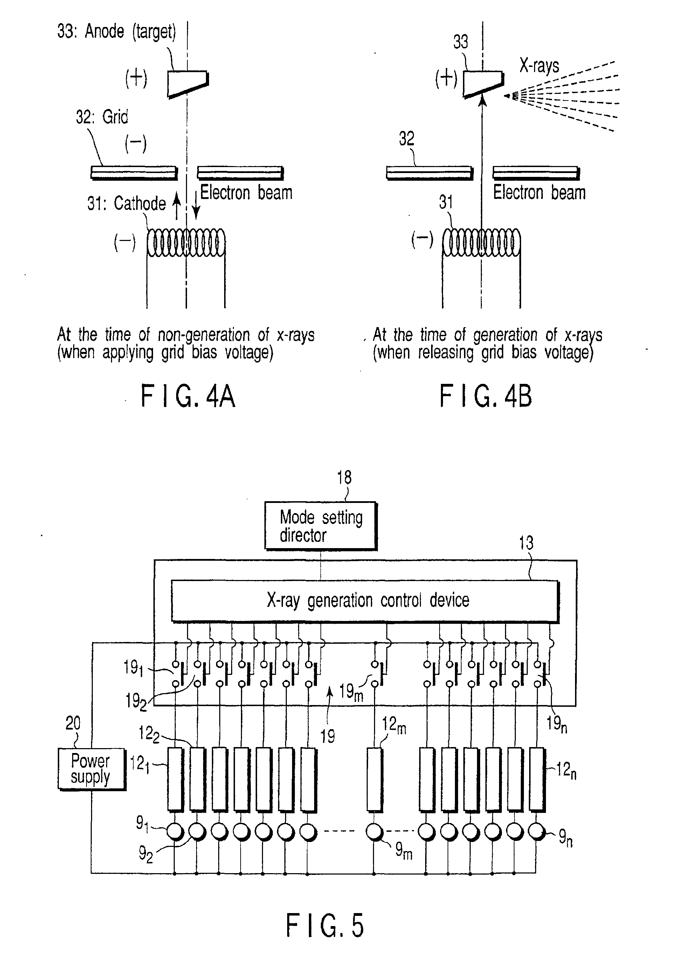

The structure of the X-ray generation device 7 will now be described in detail with reference to FIGS. 4A and 4B. Similar to a triode X-ray tube, the X-ray generation device 7 is roughly constituted by a cathode 31 (electron gun 9), an anode 33, and a grid 32. The cathode 31 is constituted by a filament which discharges thermoelectrons by heating. The anode 33 includes the target 10 with which the accelerated electrons collide, and generates X-rays by collision of the electrons. The grid 32 electrically opens/closes the path of the accelerated electrons. A high voltage is applied between the cathode 31 and the anode 33. The X-rays are generated from a part on the target plane with which the electrons collide. The surface of the anode 33 with which the X-rays collide is referred to as a focal point.

The cathode 31 has a filament obtained by stretching a material wound in a coil shape. The filament is heated by a current and discharges the thermoelectrons. The anode 33 is an electrode which sucks the electrons discharged from the filament by the high voltage applied between the cathode 31 and the anode 33, receives a shock by acceleration of the electrons to a high speed, and generates X-rays. The anode 33 is made of a material which is superior in heat conduction, e.g., copper.

Further, a tungsten plate as a target is embedded at the end of the anode 33. The surface with which the electrons converged on the target collide is a focal point. When the high-speed electrons enter a target atom and the motion of the electrons is interrupted, a part of the kinetic energy is discharged as X-rays. That is, at the time of generating the X-rays, when the electron beam 34 emitted from the cathode 31 toward the anode 33 is brought into contact with the anode 33, the sector-shaped X-rays 3 are emitted from the anode 33 in the reflecting direction according to the angle of its surface.

The X-ray generation device 7 controls emission of the electron beam from the cathode 31 to the anode 33 by arranging the grid 32 between the cathode 31 and the anode 33. That is, as shown in FIG. 4A, when the X-rays are not generated from the target 10 of the anode 33, a negative bias voltage is applied to the grid 32 so as not to pass the electron beam through the grid 32 as a gate. Furthermore, as shown in FIG. 4B, in case of generating the X-rays from the target 10 of the anode 33, the negative bias voltage is eliminated so as to allow passage of the electron beam at the grid 32. It is to be noted that the focal spot size can be miniaturized by narrowing the electron beam by using the grid 32.

As shown in FIG. 1, an image signal digitizer 14 is connected to the detector 8. This image signal digitizer 14 outputs coordinate information of the X-ray 100 detected by the detector 8 when the X-ray 100 is emitted from an arbitrary X-ray generation device 7. Incidentally, a plurality of the image signal digitizers 14 may be provided in each predetermined area of the detector 8, or one image signal digitizer 14 may have charge of the entire detector 8.

A measurement control device 15 specifies which X-rays 100 are emitted from which X-ray generation device 7 in connection with the coordinate information of the X-rays 100 output from the image signal digitizer 14, and the obtained result is recorded in a data recording device 16 together this information. When image information for one tomography is recorded in the data recording device 16, the tomogram is constituted by a data processing device 17. A monitor 21 is connected to the data processing device 17. The monitor 21 displays information such as a formed tomogram.

The detector 8 is formed into a cylindrical shape having a width in a direction along the axial line P, and many sensor arrays 8_{Lθ} are arranged in the direction L along the axial line P and the circumferential direction 8. Therefore, by emission of the X-ray from the X-ray generation device 7 for one time, detection information of the X-rays can be obtained in a range which extends along the inner surface of the cylindrical plane of the detector 8. That is, a plurality of tomograms can be obtained by the image pickup operation for one round. Moreover, it is possible to obtain a so-called X-ray radiogram obtained by emitting the X-rays from an arbitrary X-ray generation device 7 to the patient 2 for one time.

In addition, as similar to the method of constituting a given tomogram which is a two-dimensional plane based on information of a detection position by linear transmitted X-rays obtained by emitting the linear X-rays from the circumference of the measuring object, a three-dimensional stereoimage can be constituted based on information of a detection position by the planar transmitted X-rays obtained by emitting the X-rays to a predetermined area range from the circumference of the measuring object. As a result, a three-dimensional image can be constituted based on the information recorded in the data recording device 16.

FIG. 5 is a block diagram showing a control system for the X-ray generation in the X-ray CT apparatus. As shown in FIG. 1, a mode setting director 18 is provided between the measurement control device 15 and the X-ray generation control device 13. In FIG. 5, the mode setting director 18 issues a direction to the X-ray generation control device 13. Based on this, respective switch elements 19 to 19ₙ of a switch device 19 are opened/closed in accordance with the command. The switch elements 19 to 19ₙ are connected to electron gun drive circuits 12 to 12ₙ of the electron guns 9 to 9ₙ, respectively. The switch device 19 individually corresponds to each of the electron gun drive circuits 12₁ to 12ₙ, and has the switch elements 19₁ to 19ₙ connected in series to the electron gun drive circuits 12₁ to 12ₙ, respectively.

Use or non-use of each X-ray generation device 7 is selected by the X-ray generation control device 13 and the switch device 19 described above. Therefore, when the switch element 19ₘ is connected (closed) for example, power is supplied from the power supply 20, and the electron gun 9ₘ is controlled by the electron gun drive circuit 12ₘ, thereby emitting the electron beam. That is, the mode setting director 18 outputs to the X-ray generation control device 13 a direction indicative of the order to connect the switch elements 19 to 19ₙ and which of them are used. As a result, it is possible to select a serial slice mode, a single slice mode, a sector slice mode and a single shot mode or the like. Further, in each of these modes, the single ray source radiography mode or the multiple ray source synchronous radiography mode can be respectively selected.

The serial slice mode is used to synchronize a moving speed of the bed 3 of the X-ray CT apparatus 1 with an emission position of the X-rays 100 by the X-ray generation device 7 and perform image pickup in such a manner the emission position of the X-rays 100 with respect to the patient 2 can spirally shift. In this serial slice mode, therefore, it is possible to obtain a plurality of tomograms (serial slices) for the volume examination or information used for constituting a three-dimensional image.

The single slice mode is used for performing image pickup for one round by sequentially switching the emission-position of the X-rays 100 with respect to an arbitrary position of the patient 2 in the direction of the axial line P. In the single slice mode, therefore, the detector 8 has the cylindrical shape, and it is possible to obtain a tomogram at a desired position and another position adjacent thereto in the patient 2 or information used for constituting a three-dimensional image corresponding to the width of the detector 8.

The sector slice mode is used for performing image pickup at an arbitrary part of the patient 2 by sequentially switching the X-ray generation device 7 within an arbitrary angle range specified by the radiographer. At this moment, the bed 3 may be synchronously moved. Therefore, in this sector slice mode, it is possible to obtain a tomogram of a necessary part such as a part of the head, or the organ such as the heart or the stomach, or a three-dimensional image corresponding to the width of the detector 8. Furthermore, by synchronously moving the bed 3, it is possible to obtain tomograms required for the volume examination of an arbitrary part or information used for constituting a three-dimensional image of that part.

The single shot mode is used for emitting the X-rays to the patient 2 from the X-ray generation device 7 at an arbitrary angle specified by the radiographer for only one time. The detector 8 of the X-ray CT apparatus 1 according to this embodiment has the cylindrical shape. In the single shot mode, therefore, radiography similar to that using a so-called X-ray radiographic apparatus can be performed at an arbitrary angle.

Moreover, when the single ray source radiography mode is selected in each of the above-described modes by the mode setting director 18, one X-ray generation device 7 which emits the X-rays 100 is selected simultaneously in the respective modes. When the multiple ray source synchronous radiography mode is selected, a plurality of the X-ray generation devices 7 which emit the X-rays 100 are selected simultaneously in the respective modes.

Similarly, when the multiple ray source synchronous radiography mode is selected in the serial slice mode or the single slice mode, the X-rays 100 are simultaneously emitted at positions distanced at given intervals from a plurality of predetermined X-ray generation devices 7. It is preferable that a plurality of the X-ray generation devices 7 have a positional relationship such as shown in FIG. 2. In FIG. 2, X-rays 100a and 100b emitted from a plurality of the X-ray generation devices 7a and 7b do not overlap on the detector 8. In addition, illustrating a concrete example, if the number of X-ray sources is three, the setting is made in such a manner that the adjacent X-ray sources form an angle of 120°. If a number of the X-ray sources is four, the setting is made in such a manner that the adjacent X-ray sources form an angle of 90°. If the number of X-ray sources is five, the setting is made in such a manner that the adjacent X-ray sources form an angle of 72°. In this manner, the X-rays 100 are simultaneously emitted from the X-ray generation devices 7 distanced at even intervals.

Additionally, tomograms or a three-dimensional image can be constituted based on information obtained by sequentially switching a plurality of the X-ray generation devices 7 and performing image pickup.

Further, when the multiple ray source synchronous radiography mode is selected in the sector slice mode, image pickup is simultaneously carried out only at an angle designated with respect to a plurality of parts specified by the radiographer. As an instance, there is a case where image pickup of right and left kidneys is simultaneously performed.

With the above-described structure, the X-ray CT apparatus 1 rapidly performs the volume examination with respect to the patient 2 in the serial slice mode of the multiple ray source synchronous image pickup, for example. Thereafter, a part which must be subjected to a workup can be continuously rapidly examined in the sector slice mode or the single shot mode of the single ray source image pickup without moving the patient 2. In this case, the exposed dose of the X-rays emitted to obtain a tomogram used for specifying a part which must be subjected to workup can be set lower than the exposed dose of the X-rays emitted in order to obtain an X-ray transmitted image. Therefore, the total exposed dose of the patient 2 can be reduced.

Further, since the X-ray CT apparatus 1 includes the cylindrical detector 8, more tomograms can be constituted by image pickup for one time, and a three-dimensional image can be constituted based on the information of the detected X-rays. Furthermore, a stereoimage which has the three-dimensional movement can be formed by obtaining the information which is a result of repeating image pickup with respect to the same position. Consequently, further detailed examination can be performed.

Moreover, there may be a case that the information of each X-ray generation device 7 obtained by image pickup in the serial slice mode or the single slice mode is equal to the information obtained by image pickup in the single shot mode. In this case, the X-ray transmitted image with a desired angle can be obtained from the information acquired from image pickup in the serial slice mode or the single slice mode. Therefore, the total exposed dose of the patient 2 can be further reduced.

As described above, the X-ray CT apparatus 1 can obtain the tomogram of the patient 2 and the required X-ray transmitted image based on this tomogram in the examination for one time. In addition, the X-ray CT apparatus 1 does not give an excessive exposed dose to the patient 2. That is, the X-ray CT apparatus 1 can obtain desired examination information with the minimum exposed dose.

The X-ray CT apparatus according to this embodiment includes the X-ray generation control device which selects use/non-use of a plurality of the individual X-ray generation devices arranged on a concentric circle with the measuring object at the center, and the mode setting director for setting a desired operation mode with respect to the X-ray generation control device. As a result, it is possible to obtain desired measurement information in the minimum number of the tomograms while suppressing the exposed dose of the measuring object to the minimum level. Additionally, since the detector which detects the X-rays is formed into the cylindrical shape, it is possible to obtain the X-ray transmitted image by detecting emission of the X-rays for one time by using the surface.

Further, since generation of the X-rays is controlled with respect to each X-ray generation device by using the X-ray generation control device, the X-rays can be emitted by the corresponding X-ray generation devices only when necessary, thereby eliminating unnecessary emission of the X-rays. As a result, the exposed does of the measuring object can be reduced, and the thermal input to the anode of each X-ray generation device is decreased, and the continuous operating time of the anode is increased. In this manner, since a time slot in which the X-rays are not emitted is provided in each X-ray generation device, the zero point stability of the detection system can be confirmed, for example when collecting data in that time slot.

The present invention is not restricted to the foregoing embodiment, and it can be modified and carried out without departing from the scope of the invention. '

### Industrial Applicability

According to the present invention, it is possible to provide an X-ray CT apparatus and X-ray CT apparatus radiography capable of obtaining necessary examination information while suppressing the exposed dose of the measuring object to the minimum level.

## Claims

1. An X-ray CT apparatus **characterized by** comprising:
a plurality of X-ray generating means which generate X-rays, surround a measuring object, are arranged on a circle with said measuring object at the center, and are relatively movable with respect to said measuring object;
a detector which surrounds said measuring object and is arranged on a circle with said measuring object at the center, is relatively movable with respect to said measuring object, and detects X-rays emitted from said X-ray generating means and transmitted through said measuring object;
X-ray generation controlling means for selecting use/non-use of each of said X-ray generating means; and
mode setting directing means for directing a desired operation mode with respect to said X-ray generation controlling means.

2. The X-ray CT apparatus according to claim 1, **characterized in that** plural lines of said detectors are provided in a direction of relative movement of said measuring object and said X-ray generation devices.

3. The X-ray CT apparatus according to claim 1 or 2, **characterized in that** said mode setting directing means has a signal ray source radiography mode which causes said X-ray generation controlling means to switch one of a plurality of said X-ray generating means and sequentially use them.

4. The X-ray CT apparatus according to claim 1 or 2, **characterized in that** said mode setting directing means has a multiple source synchronous radiography mode which causes said X-ray generation controlling means to simultaneously switch a plurality of said X-ray generating means distanced at predetermined intervals in a circumferential direction among a plurality of said X-ray generating means and sequentially use them.

5. The X-ray CT apparatus according to claim 4, **characterized in that** said multiple ray source synchronous radiography mode simultaneously uses a plurality of arbitrary X-ray generating means whose X-rays emitted therefrom do not overlap each other on said detector.

6. The X-ray CT apparatus according to claim 1 or 2, **characterized in that** said mode setting directing means has a sector slice mode which causes said X-ray generation controlling means to switch said X-ray generating means which form a continuous part of said X-ray generating means group among a plurality of said X-ray generating means and sequentially use them.

7. The X-ray CT apparatus according to claim 1 or 2, **characterized in that** said mode setting directing means has a single shot mode which causes said X-ray generation controlling means to use one of said respective X-ray generating means for one time or simultaneously use a plurality of said X-ray generating means for one time.

8. An X-ray CT apparatus radiography **characterized by** comprising: obtaining information required for a tomogram of said measuring object by using said X-ray CT apparatus defined in any of claims 1 to 7; operating said X-ray generating means suitable for radiography of a specific part of said measuring object based on a tomogram constituted from said information; and radiographing said measuring object without changing a posture of said measuring object.
